# EUROPEAN PATENT APPLICATION

(11) **EP 1 629 834 A1**
(43) Date of publication of application: **01.03.2006**
(21) Application number: 04020431.5
(22) Date of filing: 27.08.2004
(51) Int. Cl.: A61K 9/20, A61K 31/137

(54) **Sustained release pharmaceutical composition of tolterodine**

(71) Applicant: KRKA, D.D., Novo Mesto, 8501 Novo mesto (SI)
(72) Inventor: Kramer, Andrejka, 8000 Novo mesto (SI); Pisek, Robert, 1000 Ljubljana (SI); Rajer, Tadeja, 8000 Novo mesto (SI)
(74) Representative: Becker Kurig Straus

(57) **Abstract**

The present invention is directed to a sustained release pharmaceutical composition comprising tolterodine, preferably tolterodine tartrate, to the use of said composition for the preparation of a medicament and to a method for producing such a sustained release pharmaceutical composition.

## Description

The present invention is directed to a sustained release pharmaceutical composition comprising tolterodine, preferably tolterodine tartrate, to the use of said composition for the preparation of a medicament and to a method for producing such a sustained release pharmaceutical composition.

Prolonged release formulations are highly estimated by patients as well as by physicians since they allow the possibility of reducing dosage regimens for drugs, especially in case of an oral administration. The advantages of reduced dosage regimens for the patients are an increased convenience, better assurance of compliance, reduction of severity and frequency of side effects, since prolonged release formulations maintain substantially constant blood levels and avoid the fluctuations associated with the conventional immediate release formulations administered more than once a day.

Tolterodine is a well known antimuscarinic agent specifically developed for treatment of patients with overactive bladder. Known administration forms of tolterodine are for example film-coated tablets containing 1 mg or 2 mg of tolterodine L-tartrate for immediate release in the gastrointestinal tract. These administration forms are however thought to be associated with side-effects, such as "dry mouth". In addition, tolterodine is sold commercially in extended release pharmaceutical dosage forms in order to improve patient convenience. Due to a lowering of peak drug concentrations, such a formulation is associated with reductions in the overall frequency of so-called "dry mouth", when compared to immediate release formulations.

Moreover, WO 00/27364 describes controlled release beads comprising a core unit of a substantially water-soluble or water-swellable inert material, a first layer ("sealcoat") on the core unit of a substantially water-insoluble polymer, a second layer covering the first layer and containing an active ingredient and a third layer of polymer on the second layer effective for controlled release of the active ingredient, wherein the sealcoat is adapted to control water penetration into the core. In these controlled release beads the presence of the sealcoat is of decisive importance - since in absence of such a sealcoat - formulations resulted in a longer drug release lag phase, as well as in a shorter zero order drug release phase. Additionally, due to a migration of drug into the core not all drug could get released.

What is even more, controlled release beads of the state of the art show a dissolution profile largely diverging in presence of varying ionic strength and in presence of a varying pH. As well known, the gastro-intestinal pH-value, as well as the ionic strength of the stomach and intestinal contents may largely vary. In order to provide a more reliable administration of the active substance to a patient, a lowering of intra- or inter-individual variations of pharmacokinetic parameters has to be achieved. Therefore, it has to be acknowledged that there is a need in the art to provide a sustained release composition comprising tolterodine, which composition provides a dissolution profile having a reduced dependency from the pH-value and/or the ionic strength of the dissolving medium of the sustained release composition. Moreover, a sustained release composition of the present invention does not require a time and/or cost intensive manufacturing technique and in particular does not require the application of a sealcoat in form of a substantially water-insoluble polymer onto the core element.

### In the Figures, wherein:

FIG. 1 shows a comparison of the dissolution profiles of Detrusitol®, LP-5ab and LP-6ab in phosphate buffer at a pH of 6.8.

FIG. 2 shows dissolution profiles of pellets from Detrusitol® capsules (4 mg tolterodine) in different dissolution media.

FIG. 3 shows dissolution profiles of LP-5ab capsules (4 mg tolterodine) in different dissolution media.

FIG. 4 shows dissolution profiles of LP-6ab capsules (4 mg tolterodine) in different dissolution media.

The problem of the present invention is solved by providing a sustained release pharmaceutical composition according to the invention, which comprises:
1) Inert core, a first layer, which is coated onto the inert core and comprises a tolterodine and a binder and the outer layer, which comprises hydrophobic, sustained release polymer and which is coated onto a first layer, or
2) Optionally, matrix core (consisting of e.g. a core former material, binder and tolterodine) which can be prepared directly from the powders (consisting of e.g. core former material, binder and tolterodine) and finally coated with the outer layer (which comprises hydrophobic, sustained release polymer) effective for controlled release of the active ingredient.
Said core element is either present in form of an inert core coated with a first and a second outer layer or in form of a matrix, said matrix core being a combination of a core former material, tolterodine and a binder, then only one layer-outer layer - is adapted to control the release of the drug.

In particular, the composition can be present in form of particles, such as pellets filled in capsules or compressed into tablets. These particles may have any form known in the art, for example essentially spherical, and may have a size of from 0.2 to 2.0 mm, preferably of from 0.5 to 1.5 mm, more preferably of from 0.7 to 1.2 mm. The size of these particles can be determined by means of sieving analyses. Techniques for filling particles into capsules or compressing to tablets are known in the art.

Multiparticulates, in particular such as described before, have numerous therapeutic advantages over single unit administration forms. When taken orally, multiparticulates generally disperse freely in the gastrointestinal tract, maximize absorption, minimize side effects and reduce inter-and intra-patient variability.

The layers of the sustained release composition, namely the first layer, the outer layer, the separating layer and further optional layer(s) may be applied on to the cores according to techniques known in the art and in particular using a fluidized bed or a coating pan. Preferably a fluidized bed coater is used, e.g. a bottom, a tangential or a top-spray coater. A fluidized bed system, e.g. from Wurster or Huettlin, is one in which an air jet injected from underneath, fluidizes the particles and effects a drying while the coating material is sprayed on the cores.

To create pellets directly from powders and further film coating apply on it, rotor (tangential spray fluidized bed) system can be used. This system is a combination of fluid bed system fitted with a solid rotating disc at the base.

The discrete coated particles, e.g. granules or pellets, thus obtained can be filled into larger units, such as capsules or formed into tablets according to any technique known in the state of the art.

Both processes described above are very efficient and economical reasonable because all phases of the manufacturing, such as coating and drying (in case of rotor technology also core formation), take place in a single pot, e.g. a fluidized bed coater. Further, such a production process leads to compositions wherein the reproducibility of the release characteristics of tolterodine is excellent.

Tolterodine tartrate can be prepared according to the process described in EP 325 571 or EP 667 852, EP 960 109, WO 01/49649, WO 03/14060, WO 98/03067, CN 1379018 or as described in article Andersson, et al., Asymmetric Total Synthesis of (+)-*Tolterodine,* a New Muscarinic Receptor Antagonist, via Copper-Assisted Asymmetric Conjugate Addition of Aryl Grignard Reagents to 3-Phenyl-prop-2-enoyl-oxazolidinones, Journal of Organic Chemistry, vol. 63, p. 8067.8070 (1998).
Various types of particle size of tolterodine can be used according to the invention, preferably tolterodine with the particle size from 5 to 200 µm is used.
Tolterodine used in the formulation according to the invention can be present in different form of a salt thereof. Examples of useful salts are the addition salts with an inorganic acid selected from hydrochloric, hydrobromic, phosphoric, nitric and sulfuric acid or with an organic acid selected from acetic, propanic, hydroxyacetic, oxalic, lactic, pyruvic, malonic, succinic, fumaric, malic, glutaric, maleic, citric, cyclamic, sulphonic, benzensulphonic, pamoic and the like.
Examples of useful salts are also described in WO 03/90734.

The term "tolterodine" as used in the present application comprises tolterodine (*N,N*-diisopropyl-3-(2-hydroxy-5-methylphenyl)-3-phenylpropanamine), as well as any salt of tolterodine with pharmaceutically acceptable organic or inorganic acid(s), in particular with tartaric acid, namely tolterodine tartrate, and encompasses compounds of all stereo configurations, i.e. as well naturally occurring and non-naturally occurring stereo configurations. For reasons of its long established use as a pharmaceutically active substance, in particular tolterodine [(R, R)-tartrate] is preferred.

The composition preferably comprises tolterodine, calculated as tolterodine tartrate, in an amount of from 0.5 to 10.0 wt %, preferably of from 1.0 to 5.0 wt % and more preferably of from 1.0 to 3.0 wt % and especially of from 1.5 to 2.5 wt %, each on basis of the total weight of the sustained release composition. The corresponding amounts of tolterodine and other tolterodine salts for further compositions may be easily obtained by calculation on basis of the above-indicated values relating to tolterodine tartrate.

Of course, when desired, also dosage forms permitting a less frequent administration, such e.g. every 36 h or 48 h can be prepared by a skilled person.

The composition can comprise at least one sustained release polymer (such as for example neutral polymers of ethyl acrylate/methyl methacrylate, ethylcellulose, polyvinyl acetate (PVAc) and polyvinyl pyrrolidone (PVP) or mixtures thereof) in an amount of preferably 2 to 35 wt %, more preferably 3 to 30 wt %, and most preferably 4 to 25 wt %, each on basis of the total weight of the sustained release composition.

A sustained release polymer can be present in the outer layer which can be chosen by a skilled person on basis of the general knowledge in the art. Surprisingly advantageous results can be obtained when the sustained release polymer in the outer layer is a neutral polymer of ethyl acrylate/methyl methacrylate, preferably Eudragit NE 30 D, ethylcellulose, or a mixture of polyvinyl acetate (PVAc) and polyvinyl pyrrolidone (PVP) (applied for example in form of a pre-formulated dispersion of PVAc and PVP), or mixtures thereof. The selection of the above-mentioned sustained release polymers together with the composition of the outer layer and its thickness provide a particularly reliable and advantageous dissolution/liberation profile of the active compound tolterodine at neutral and/or acidic pH-ranges.

The thickness of the outer layer can be in the range of from 10 to 40 µm, preferably of from 15 to 30 µm and more preferably of from 20 to 30 µm. The sustained release polymers can be employed in the form of an aqueous suspension or in the form of a solution in an organic solvent during the application process.

The outer layer can also comprise a plasticizer for further enhancing the film formation and its flexibility. Suitable plasticizers can be chosen according to the general knowledge in the art by a skilled person and are for example 1, 2-propylene glycol, triethyl citrate, polyethylene glycol and triacetin, with triethyl citrate being the preferred plasticizer. It is preferred that the amount of plasticizer ranges from 0 to 30 wt %, preferably from 0.5 to 19 wt.%, based on the dry weight of the sustained release polymer(s) as defined above.

In the outer layer an antisticking agent, such as talc, can also be used. The weight ratio of antisticking agent to the sustained release polymer(s) can be chosen by a skilled person according to the general knowledge in the art. In particular, the weight ratio of antisticking agent to the sustained release polymer can be of from 1:1 to 1:10 (wt. / wt.), preferably from 1:1 to 1:5 (wt. / wt.).

The thickness of the outer layer controls the release rate of tolterodine from the sustained release composition. In the context of the present invention, the term release rate means the amount of tolterodine which is liberated from the composition in a given period of time. Initially, water has to penetrate through the outer layer and enter the first layer and/or the core in order to dissolve the pharmaceutically active substance tolterodine before said tolterodine may diffuse through the outer layer. As should be clear, the thickness and/or composition of the outer layer has to be adapted to the chemical and/or physical properties of tolterodine, such as for example to its molecular size, dissolution properties, in order to obtain an optimal sustained release property of the pharmaceutical composition. The present inventors found that the present pharmaceutical composition, in particular the outer layer structure surprisingly assures desired release of tolterodine as the pharmaceutically active substance. For currently preferred applications, tolterodine is the sole pharmaceutically active compound (apart of formulation agents such as binders, coloring agents, etc.) in the sustained release composition.

The release rate of tolterodine decreases with increasing thickness of the outer layer. However, also a minimal amount of outer layer has to be observed when preparing a sustained release composition according to the present invention. The coverage provided by the outer layer is preferably not less than 1.0 mg/cm², which corresponds to a thickness of about 10 µm, since otherwise for example an uncontrolled fast release of tolterodine may occur. In particular, otherwise film defects and burst effects could occur. The dissolution profiles of pellets according to the present invention are shown in FIG.s 1, 3 and 4 and are very advantageous since they permit for example a release of at least 50 % but less than 90% after a dissolution time of 5 hours at a pH between 1.2 to 6.8 under simulated release conditions. Such a dissolution profile is highly desired since it provides a release of the active substance tolterodine in the respective target regions of the gastro-intestinal tract in well-balanced amounts.

Besides the release rate, also the lag time is influenced by the thickness of the outer layer. In the context of the present application, the term lag time means the time between first contact of the pharmaceutical composition with water and the first measurable release of tolterodine. With increasing thickness of the first layer the lag time is also increased.

When formulating a sustained release composition on basis of tolterodine, in particular tolterodine tartrate, for a once daily administration, the first layer provides a coverage of from 1 to 5 mg/cm², preferably of from 1 to 3 mg/cm². The coverage of the first layer can be determined readily by a skilled person by an electron microscope.

An important advantage of the sustained release compositions of the present is that they do not require the application of a seal coat on the inert core, such as e.g. a layer of a substantially water-insoluble polymer, or in other words, the sustained release composition of the present invention advantageously provides an inert core free of or not in direct contact with a layer of a substantially water-insoluble polymer.

In particular, the core element of a pharmaceutical composition according to the invention can comprise an inert core, which is provided with a first (inner) layer comprising tolterodine and a binder, said second layer being layered on said inert core.

The term "inert core" pertains a core made of any pharmaceutically acceptable material which may be formed to particulates and essentially does not induce a degradation of tolterodine (i.e. which does not induce an increase of the degradation of tolterodine of more than for example 5 %, preferably not more than 2 %) or even enhances the stability of tolterodine, when compared to the same tolterodine compound stored as such under identical storage conditions (e.g. during three months after production at 20 °C).

These inert core(s) can be made from pharmaceutically acceptable monosaccharides, disaccharides, oligosaccharides, polysaccharides and mixtures thereof, such as saccharose and starch or cellulose, such as microcrystalline cellulose. In particular, the inert core can consist of not less than 62.5% and not more than 91.5% sucrose, the remainder is starch. The pharmaceutical composition can comprise inert core(s) in an amount of preferably from 40 to 90 wt %, more preferably of from 55 to 85 wt %, and most preferably of from 60 to 80 wt %, each on basis of the total weight of the sustained release composition.

These inert core(s) are provided with a first (inner) layer as illustrated on more detail below comprising tolterodine and a binder. This binder can be any pharmaceutically acceptable binder known in the art. For example, the second layer can be selected from the group consisting of cellulose derivatives and PVP (Polyvinyl pyrrolidone). Preferred cellulose derivatives are hydroxypropylmethyl cellulose and/or hydroxypropyl cellulose. The amount of binder in the first layer and/or the matrix is preferably of from 2 to 5 wt %, more preferably of from 3 to 5 wt %, and most preferred of from 3 to 4 wt %, each on basis of the total weight of the pharmaceutical sustained release composition.

The weight ratio of tolterodine to binder in the first layer and/or the matrix can be for example of from 2 : 1 (wt. / wt.) to 1 : 5 (wt. / wt.), preferably of from 1 :1 (wt. / wt.) to 1 : 3 (wt. / wt.), each calculated on the basis of tolterodine tartrate.

The first and second outer layer can also comprise an antisticking agent. Such an antisticking agent will be recommendable if higher concentrations of binder are used. An antisticking agent, such as talc, reduces the sticking tendency and thereby prevents the agglomeration of pellets, as well as adhesion effects to the wall during the coating process.

As a material for forming a structured particle - matrix pellets (from the powders) that is not easily disintegrated in the gastrointestinal tract, microcrystalline cellulose is used. It is preferred that the weight ratio of it in the formulation is over 30%, more preferred between 50 and 60%. Pellets prepared with microcrystalline cellulose and tolterodine are insufficiently strong and also provide an insufficiently controlled release of the active substance tolterodine. To improve retarding effect, the same polymers as used for coating can be used in the granulating step (neutral polymers of ethyl acrylate/methyl methacrylate, ethylcellulose, or mixture of PVAc and PVP as a separate polymer or mixtures of these polymers). Optionally, these polymers can be used in further outer coating process(es) to assure desired release of tolterodine from the pellets.

The matrix core formulation is a consisted of matrix core material, tolterodine and a binder. In this case, all materials are combined, e.g. by mixing, binding and drying step to form a matrix core formulation. In particular, a matrix pellet can be prepared wherein tolterodine and matrix core material, e.g. microcrystalline cellulose is pelletized with hydrophobic polymer-ethylcellulose or polymethacrylate or PVAc/PVP and optionally coated with the same or different polymer which is controlling drug release.

Further ingredients known in the art of formulating pharmaceutical compositions can be included either in the matrix core formulation and/or in one of the layers described above and/or be provided in form of an additional layer (apart of the fact that no substantially water-insoluble polymer is applied onto the inert carrier core). For example, coloring agents, sucrose, lactose, pharmaceutically grade surfactants and/or flavoring agents can be added.

The sustained release compositions according to the present invention can be used for the preparation of a medicament for the treatment or prevention of urge incontinence and other symptoms of unstable or overactive urinary bladder.

As lined out in detail before, a sustained release composition according to the present invention can be adapted to be administered one or several times per day or can be administered every second, third, fourth, sixth, seventh day, etc.. In order to increase the patient compliance, in particular a once daily administration form is desired.

The sustained release composition of the present invention can be administered preferably in an peroral administration form.

According to another aspect, the present invention also provides a method for the manufacture of a tolterodine sustained release pharmaceutical composition, said sustained release pharmaceutical composition having a first layer comprising tolterodine and a binder, which first layer is coated on an inert core and second layer comprising a hydrophobic sustained release polymer, which second layer is coated on the first layer. The process comprises the steps: coating a first layer on a inert core, wherein said coating and drying steps being performed in the same container at the same time; coating the second layer onto the first layer, wherein said coating and drying steps being performed in said same container at the same time.

Under manufacturing technical aspects it can be advantageous when said coating and drying steps of the first layer and said coating and drying steps of the second layer are performed in the same container.

The method for the manufacture of a composition according to the present invention is advantageous, under aspects of manufacture technique, as well as under the aspects of production costs and production time, because the phases of the process, i.e. coating and drying steps, as well as the forming of the second and the first layer, can take place in the same container. By this simple arrangement, surprisingly the reproducibility of the process and thereby the quality of the resulting pharmaceutical composition is excellent.

In the case of rotor technology inert cores are not required. In this case, all materials are combined, e.g. by mixing, binding, and drying step to form a matrix core formulation. In particular, a matrix pellet can be prepared wherein tolterodine and a matrix core material, e.g. microcrystalline cellulose is palletized with hydrophobic polymer-ethylcellulose or polymethacrylate and optionally coated with the same or different polymer which is controlling drug release. It is also one pot system, less time consuming than process mentioned above, but it is preferably used for high dose system.

The present invention provides thus an advantageous additional sustained release composition which provides an improved dissolution property with a reduced dependence from the ionic strength and the pH-value of the medium used for dissolving the sustained release composition of the present invention. This will provide significant advantages in terms of lower intra- or inter-individual variations of pharmacokinetic parameters. Moreover, the sustained release composition does not require a time and/or cost intensive manufacturing technique, and in particular does not require an application of a sealcoat in form of a substantially water-insoluble polymer onto the core element.

The invention is illustrated in more detail by the following non-limiting examples.

### EXAMPLES

### Example 1

### Sustained release composition LP-5ab

65.0 g of hydroxypropyl cellulose was dissolved in 500 g of purified water. 75.0 g of talc was suspended in 300 g of purified water. 60.0 g of tolterodine tartrate was dispersed in 1200 g of purified water and heated on 40°C. Than, the solution of hydroxypropyl cellulose and suspension of talc were added during the stirring to obtain a substantially homogeneous dispersion. This dispersion was then sprayed onto 2385 g of inert carrier cores, i.e. non pareil seeds, in a Wurster fluidized bed equipment to form carrier cores with a second layer. To avoid settling, stirring was continued throughout the coating process.

2412,62 g of the coated cores were additionally coated in the Wurster fluidized bed equipment with the dispersion made of 804.17 g of Eudragit NE 30 D (30% dispersion of ethyl acrylate/methyl methacrylate copolymer), 241.25 g of talc and 884,58 g of purified water.

### Example 2

### Sustained release composition LP- 6ab

65.0 g of polyvinyl pyrrolidone (PVP) was dissolved in 500 g of purified water. 75.0 g of talc was suspended in 300 g of purified water. 60.0 g of tolterodine tartrate was dispersed in 1200 g of purified water and heated on 40°C. Than, the solution of polyvinyl pyrrolidone and suspension of talc were added during the stirring to obtain a substantially homogeneous dispersion. This dispersion was then sprayed onto 2385 g of inert carrier cores, i.e. non pareil seeds, in a Wurster fluidized bed equipment to form carrier cores with a second layer. To avoid settling, stirring was continued throughout the coating process.

2412,62 g of the coated carrier cores were additionally coated in the Wurster fludized bed equipment with the dispersion made of 1447.52 g of Surelease (25% dispersion, consisting primarily of ethylcellulose plasticized with fractionated coconut oil) and 965.08 g of purified water.

### Example 3

### Sustained release composition LP-4

1836.0 g of microcrystalline cellulose and 54.0 g tolterodine tartrate were mixed together in a rotor granulator during the spraying process with dispersion made of 1500 g of Eudragit NE 30 D and 1800 g of purified water. The particles obtained were spheres having particle sizes of 0.2 to 1.0 mm.

2112,62 g of the matrix cores were additionally coated in the rotor granulator with the dispersion made of 268.17 g of Eudragit NE 30 D (30% dispersion of ethyl acrylate/methyl methacrylate copolymer), 80.42 g of talc and 295,00 g of purified water.

### Example 4

### Dissolution Tests

The pellets corresponding to 4 mg of tolterodine tartrate according to example LP-5ab and LP-6ab or the state of the art pellets from the Detrusitol® capsules were subjected to a dissolution vessel test. The dissolution test was carried out using USP apparatus I (USP 27, page 2303) in media at 100 rpm in three different dissolution media (900 ml) at 37°C:
- phosphate buffer (USP 27, page 2724) having pH of 6.8 (ionic strength of the buffer is 0.083 mol/L) calculated in accordance with the literature-Quantitative Chemical Analysis written by Daniel C. Harris, age 175)
- phosphate buffer (USP 27, page 2724 with the additional of 0.22 mol/L KCl) having pH of 6.8 (ionic strength of the buffer is 0.524 mol/L calculated in accordance with the literature-Quantitative Chemical Analysis written by Daniel C. Harris, page 175). This phosphate buffer provides a relatively higher ionic strength, when compared to the before-mentioned phosphate buffer (USP);
- 0.1 M (mol/L) HCl, pH=1.2

The dissolution has been monitored by HPLC, equipped with UV detector at 205 nm.

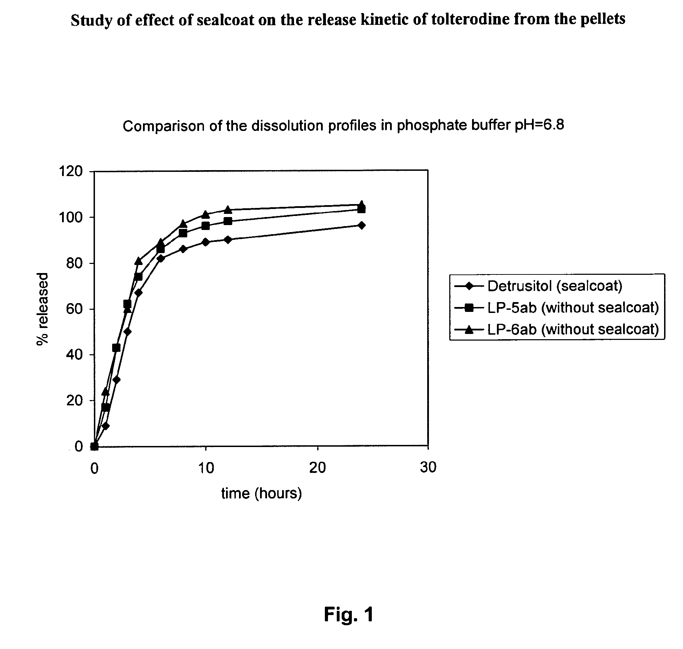

FIG. 1 shows a comparison of the dissolution profiles in phosphate buffer (having at the beginning of the test pH = 6.8) between pellets of a pharmaceutical composition of the state of the art (Detrusitol®) comprising 4 mg of tolterodine tartrate and provided with a sealcoat on the pellet core, and pellets comprising 4 mg of compositions LP-5ab and LP-6ab according to the present invention. As can be seen from FIG. 1, the pellets comprising 4 mg of compositions LP-5ab and LP-6ab without sealcoat show very similar release profile of tolterodine tartrate when compared to the pellets of composition Detrusitol® provided with a sealcoat. Our results prove in contrast to the state of the art that sealcoat is not of decisive importance to achieve desired dissolution profile (formulation without sealcoat does not show a lag phase, or shorter zero order drug release phase and total drug releases from the core).

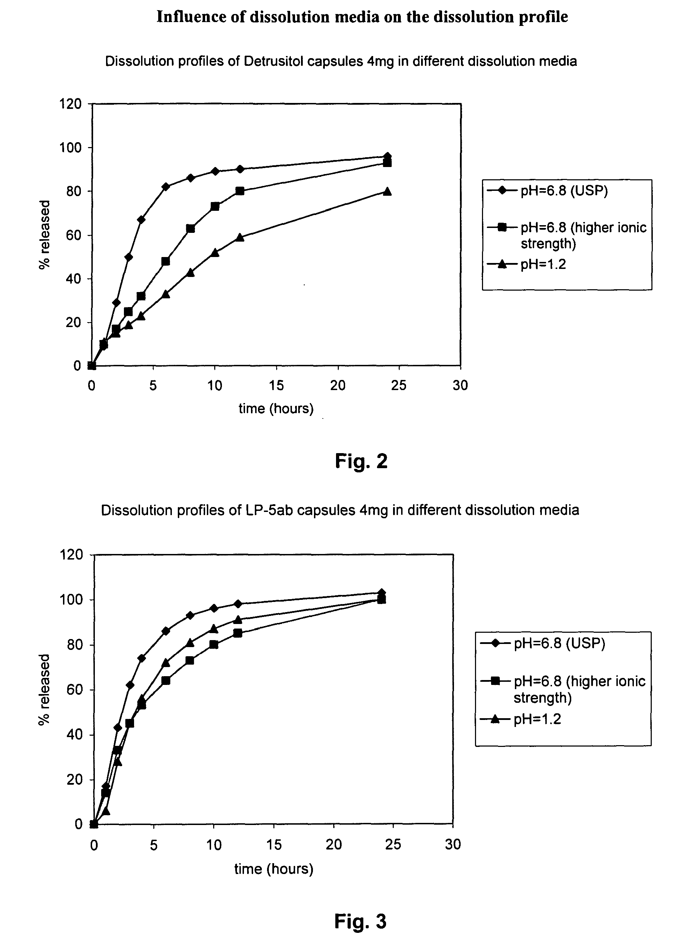

FIG. 2 shows that pellets of Detrusitol® comprising 4 mg of tolterodine [(R,R)-tartrate)] have a largely decreased dissolution either at a lower pH (pH = 1.2) or at a higher ionic strength. The dissolution properties will thus largely vary depending on the physiological conditions present in the mammal or human having ingested said sustained release composition.

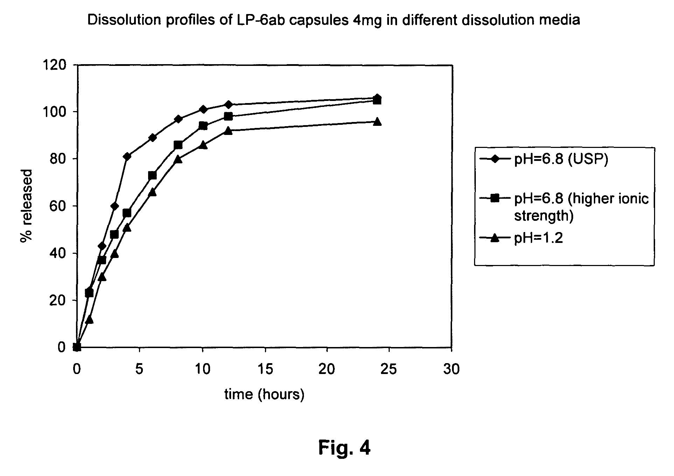

Fig. 3 and FIG. 4 show that the pellets comprising 4 mg of compositions LP-5ab and LP-6ab provide a less diverging dissolution profile either in presence of a medium having a higher ionic strength or a low pH.

## Claims

1. A sustained release pharmaceutical composition, comprising
An outer layer coated on the first layer or on the matrix core, which outer layer comprises a hydrophobic sustained release polymer and,
at lease one core element, which is selected from
(i) an inert core, said inert core being provided with a first layer comprising tolterodine and a binder, and
(ii) a matrix core formulation, said matrix core formulation being a combination of a matrix core material, tolterodine and a binder.

2. The sustained release pharmaceutical composition according to claim 1, which is in the form of particles, which are preferably filled in capsules or compressed in tablets.

3. The sustained release pharmaceutical composition according to any of the preceding claims, which comprises tolterodine in an amount of from 0.5 to 10.0 wt %, preferably of from 1.0 to 5.0 wt % and especially of from 1.5 to 2.5 wt %, each calculated as the salt form.

4. The sustained release pharmaceutical composition according to any of the preceding claims, wherein the hydrophobic sustained release polymer selected from the group consisting of neutral polymers of ethyl acrylate/methyl methacrylate, ethylcellulose, and polyvinyl acetate (PVAc) and polyvinyl pyrrolidone (PVP))

5. The sustained release pharmaceutical composition according to any of the preceding claims, wherein
the amount of the hydrophobic sustained release polymer is in the range of from 2 to 35 wt %, preferably 4 to 25 wt % and/or
wherein the amount of the carrier cores is in a range of from about 40 to 90 wt %, preferably 60 to 80 wt % and/or
wherein the amount of the binder is in the range of 2 to 5 wt %, preferably 3 to 4 wt %, based on the pharmaceutical composition.

6. The sustained release pharmaceutical composition according to any of the preceding claims, wherein the weight ratio of tolterodine to binder is of from at least 2 : 1 and up to 1 : 5, preferably of from 1 : 1 to 1 : 3, each calculated on the basis of tolterodine tartrate.

7. The sustained release pharmaceutical composition according to any of the preceding claims, wherein the coverage by the first layer is preferably not less than 1.0 mg/cm², which corresponds to a thickness of about 10 µm.

8. Use of a sustained release composition according to any of the preceding claims for the preparation of a medicament for the treatment or prevention of urge incontinence and other symptoms of unstable or overactive urinary bladder.

9. The use according to claim 8, wherein said sustained release composition is administered once-daily.

10. A process for the manufacture of a sustained release pharmaceutical composition according to any of the claims 1 to 7, comprising the steps:
coating and drying the first layer on an inert core element,
coating and drying a second layer to the first layer of the particles,
said coating and drying steps being performed in said same container.

11. The process according to claim 10, comprising the steps:
preparation of the matrix core from the powders,
further coating wherein the matrix core preparation and coating are performed by means of rotor technology.
